(19) Europäisches Patentamt
European Patent Office
Office européen des brevets

(11) Numéro de publication: **0 050 563 B1**

# (12) FASCICULE DE BREVET EUROPEEN

(45) Date de publication du fascicule du brevet: **09.05.84**

(51) Int. Cl.³: **C 07 D 471/04**, A 61 K 31/44 // (C07D471/04, 235/00, 221/00)

(21) Numéro de dépôt: **81401606.9**

(22) Date de dépôt: **15.10.81**

(54) **Dérivés d'imidazo(1,2-a)pyridine, leur préparation et leur application en thérapeutique.**

(30) Priorité: **22.10.80 FR 8022537**

(43) Date de publication de la demande: **28.04.82 Bulletin 82/17**

(45) Mention de la délivrance du brevet: **09.05.84 Bulletin 84/19**

(84) Etats contractants désignés: **AT BE CH DE FR GB IT LI LU NL SE**

(56) Documents cités:
**GB - A - 1 076 089**

**JOURNAL OF MEDICINAL CHEMISTRY, vol. 12, no. 1, janvier 1969, WASHINGTON (US) L. ALMIRANTE et al.: "Derivatives of imidazole. III. Synthesis and pharmacological activities of nitriles, amides and carboxylic acid derivatives of imidazo (1,2-alpha)pyridine", pages 122-126**

(73) Titulaire: **SYNTHELABO, 58, rue de la Glacière, F-75621 Paris Cedex 13 (FR)**

(72) Inventeur: **Kaplan, Jean-Pierre, 20, rue Arnoux, F-92340 Bourg-la-Reine (FR)**
Inventeur: **George, Pascal, 39, rue Henri de Vilmorin, F-94400 Vitry S/Seine (FR)**

(74) Mandataire: **Thouret-Lemaitre, Elisabeth, Service Brevets - SYNTHELABO 58, rue de la Glacière, F-75621 Paris Cedex 13 (FR)**

EP 0 050 563 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des dérivés d'imidazo[1,2-a]pyridine, leur préparation et leur application en thérapeutique.

Des imidazo[1,2-a]pyridines ont déjà été décrites dans la littérature, par exemple dans les brevets britanniques 991 589 et 1 076 089 et dans diverses publications.

Les composés de la présente invention répondent à la formule (I)

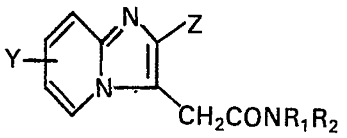

(I)

dans laquelle

Y représente un atome d'hydrogène ou d'halogène ou un radical $C_{1-4}$ alkyle,

Z représente un radical naphthyle ou un radical

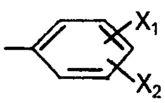

dans lequel $X_1$ et $X_2$ sont chacun indépendamment l'un de l'autre, un atome d'hydrogène ou d'halogène, un radical $C_{1-4}$ alcoxy, un radical $C_{1-6}$ alkyle, le groupe $CF_3$, $CH_3S$, $CH_3SO_2$ ou $NO_2$,

$R_1$ et $R_2$ représentent chacun indépendamment l'un de l'autre soit un atome d'hydrogène, soit un radical $C_{1-5}$ alkyle droit ou ramifié pouvant porter un ou plusieurs atomes d'halogène, un radical hydroxy, $N(C_{1-4}\ alkyl)_2$, carbamoyle ou $C_{1-4}$ alcoxy, soit le radical allyle, soit le radical propargyle, soit un radical $C_{3-6}$ cycloalkyle, soit le radical benzyle, soit le radical phényle,

$R_1$ et $R_2$ ne pouvant être tous deux des atomes d'hydrogène ou bien

$NR_1R_2$ représentent ensemble un hétérocycle comportant de 3 à 6 atomes de carbone, ou un hétérocycle de formule 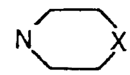 dans laquelle X est O,S,CHOR' ou N–R, R' étant un atome d'hydrogène ou le radical benzyle et R étant un atome d'hydrogène, un radical $C_{1-4}$ alkyle ou le radical phényle pouvant porter un radical méthoxy ou un atome d'halogène.

Les composés préférés de l'invention sont ceux dans lesquels $R_1$ et $R_2$ sont tous deux des radicaux alkyles.

Parmi ceux-ci le choix se porte sur les composés dans lesquels Y est en position 6 et représente soit un atome d'halogène, soit le radical méthyle.

Et enfin on peut citer parmi ces derniers les composés dans lesquels Z est un radical 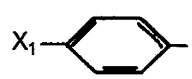 dans lequel $X_1$ est un atome d'halogène ou le radical $CH_3$.

Selon l'invention, on peut préparer les composés (I) selon le schéma réactionnel suivant:

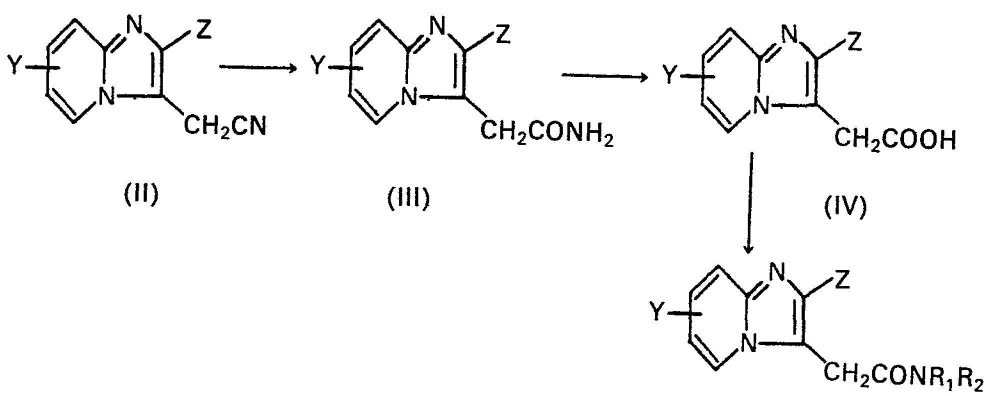

(II) (III) (IV) (I)

La réaction de transformation du nitrile (II) en amide primaire est effectuée selon une méthode classique par exemple à l'aide d'un acide, tel que l'acide chlorhydrique sec gazeux, dans un solvant tel que l'acide formique à une température allant de 15 à 50 °C.

La saponification de l'amide primaire (III) en acide (IV) se fait dans de la potasse éthanolique à la température du reflux.

L'amidification de l'acide (IV) en composé (I) est réalisée selon toute méthode appropriée, par exemple par réaction de l'acide (IV) avec l'amine $HNR_1R_2$ en présence de carbonyldiimidazole ou par réaction du chlorure de l'acide (IV) avec l'amine $HNR_1R_2$.

La méthode générale de préparation des nitriles de départ (II) est décrite dans la littérature, en particulier dans le brevet britannique 1 076 089.

Les exemples suivants illustrent la présente invention. Les analyses et les spectres IR et RMN confirment la structure des composés.

### Exemple 1

Chloro-6-N,N-diméthyl(chloro-4-phényl)-2-imidazo[1,2-a]pyridine-3-acétamide.

[ Y = Cl-6, Z = 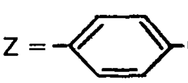 -Cl, $R_1$ = $R_2$ = $CH_3$ ]

1. On ajoute 22 g (0,0788 mole) de chloro-6-(chloro-4-phényl)-2- imidazo[1,2-a]pyridine-3-acétonitrile à 85 ml d'acide formique à 99%, on

traite la solution par un courant d'acide chlorhydrique sec gazeux, pendant 3 à 4 heures. Quand tout le nitrile est transformé, on chauffe un peu la solution afin de la dégazer puis on verse la solution refroidie dans 1 l d'eau; on agite 10 minutes puis on alcalinise avec 200 ml d'ammoniaque concentrée. On filtre le solide, le lave abondamment à l'eau et le sèche à la trompe d'eau. On fait recristalliser, dans de l'éthanol, le chloro-6-(chloro-4-phényl)-2- imidazo[1,2-a]pyridine-3-acétamide.

F = 285–7 °C

2. A 550 ml d'éthanol à 75%, on ajoute successivement 19,2 g de chloro-6-(chloro-4-phényl)-2-imidazo[1,2-a]pyridine-3-acétamide et 19 g de KOH. La suspension est portée à la température du reflux pendant 10–16 heures. La réaction terminée, la solution est concentrée sous vide et le résidu est dissous dans 1/2 litre d'eau. Le léger insoluble est filtré et le filtrat est traité par 50 ml d'acide acétique. L'acide attendu précipite, on le filtre et le sèche grossièrement. On reprend le produit brut par 500 ml d'acétone et filtre à chaud l'acide chloro-6-(chloro-4-phényl)-2-imidazo[1,2-a]pyridine-3-acétique.

F = 258–260 °C

3. On met en suspension dans 60 ml de tétrahydrofuranne sec 4 g (12,45 mmoles) d'acide chloro-6-(chloro-4-phényl)-2-imidazo[1,2-a]pyridine-3-acétique et 2,42 g (14,94 mmoles) de carbonyldiimidazole. On agite le mélange réactionnel à 20 °C jusqu'à la fin du dégagement de gaz carbonique puis on chauffe légérement à 40 °C pendant 15 minutes et on refroidit à 0 °C. On ajoute alors 14,94 mmoles de diméthylamine en solution dans 5 ml de tétrahydrofuranne. On agite la suspension pendant 15 minutes à 20 °C puis on la concentre; on traite le résidu par 300 ml d'eau et 50 ml d'une solution aqueuse saturée de $NaHCO_3$. On filtre l'insoluble, lave à l'eau et sèche. On fait recristalliser le composé obtenu dans un solvant tel que l'éthanol.

F = 230 °C

Exemple 2

Méthyl-4 [(chloro-4-phényl) -2- imidazo-[1,2-a]pyridinyl-3] méthylcarbonyl -1-pipérazine.

[Y = H, Z = 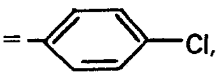, $NR_1R_2$ = 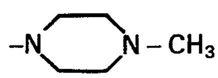

On ajoute 4,5 g (15,64 mmoles) d'acide (chloro-4-phényl)-2-imidazo[1,2-a]pyridine-3-acétique à une suspension de chlorure de N,N-diméthyl-chloro-méthylèneiminium, préparé par addition de 2,2 g (17,75 mmoles) de chlorure d'oxalyle à 30 ml de diméthylformamide (DMF) à −10 °C. On agite la suspension pendant 15 minutes à 0 °C puis on y ajoute, peu à peu, à 0 °C, 5,4 g (54 mmoles) de méthyl-4-pipérazine en solution dans 10 ml de DMF sec. On agite la solution pendant 8 heures puis on la verse dans 750 ml d'eau. On extrait l'amide avec du $CH_2Cl_2$, on sèche la phase organique sur $Na_2SO_4$, on la concentre et on fait passer le résidu sur colonne de silice (éluant $CH_2Cl_2/CH_3OH$ 9/1) puis on fait recristalliser le composé obtenu dans un mélange éther isopropylique/acétonitrile.

F = 175 °C

Tableau

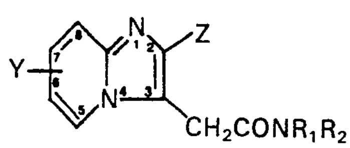

| Composé | Y | Z | $NR_1R_2$ | F(°C) |
|---|---|---|---|---|
| 1 | H | 4-Cl–$C_6H_4$ | $NHCH_3$ | 234 |
| 2 | H | 4-Cl–$C_6H_4$ | $N(CH_3)_2$ | 179 |
| 3 | H | 4-Cl–$C_6H_4$ | 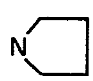 | 187–8 |
| 4 | H | 4-Cl–$C_6H_4$ | 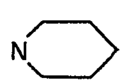 | 190 |
| 5 | H | 4-Cl–$C_6H_4$ | 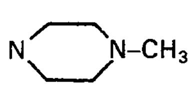 | 175 |
| 6 | H | 3-$CF_3$–$C_6H_4$ | 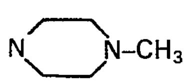 | 157,5–158 |

Tableau (suite 1)

| Composé | Y | Z | $NR_1R_2$ | F(°C) |
|---|---|---|---|---|
| 7 | H | 4-Cl–$C_6H_4$ | 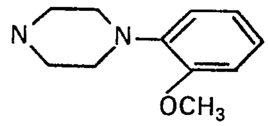 | 206–7 |
| 8 | H | 4-Cl–$C_6H_4$ | 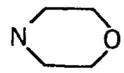 | 242 |
| 9 | 6-Cl | 4-Cl–$C_6H_4$ | $NHCH_3$ | >290 |
| 10 | 6-Cl | 4-Cl–$C_6H_4$ | $NHC_2H_5$ | 280–2 |
| 11 | 6-Cl | 4-Cl–$C_6H_4$ | NH–n–$C_3H_7$ | 229–30 |
| 12 | 6-Cl | 4-Cl–$C_6H_4$ | NH–i–$C_3H_7$ | 259 |
| 13 | 6-Cl | 4-Cl–$C_6H_4$ | NH–n–$C_4H_9$ | 225 |
| 14 | 6-Cl | 4-Cl–$C_6H_4$ | NH–t–$C_4H_9$ | 224 |
| 15 | 6-Cl | 4-Cl–$C_6H_4$ | 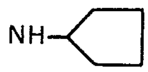 | 243–5 |
| 16 | 6-Cl | 4-Cl–$C_6H_4$ | $NHC_6H_5$ | 265–7 |
| 17 | 6-Cl | 4-Cl–$C_6H_4$ | $NHCH_2C_6H_5$ | 253–4 |
| 18 | 6-Cl | 4-Cl–$C_6H_4$ | $NHCH_2CH_2OH$ | 260–1 |
| 19 | 6-Cl | 4-Cl–$C_6H_4$ | $NHCH_2CH_2OCH_3$ | 197 |
| 20 | 6-Cl | 4-Cl–$C_6H_4$ | $NHCH_2CH_2N(CH_3)_2$ | 199–201 |
| 21 | 6-Cl | 4-Cl–$C_6H_4$ | $NHCH_2CH=CH_2$ | 233 |
| 22 | 6-Cl | 4-Cl–$C_6H_4$ | $NHCH_2–C{\equiv}CH$ | 239 |
| 23 | 6-Cl | 4-$CH_3$–$C_6H_4$ | $NHC_2H_5$ | 238 |
| 24 | 6-Cl | 4-Cl–$C_6H_4$ | $NHCH_2CF_3$ | 258 |
| 25 | 6-Cl | 4-Cl–$C_6H_4$ | $NHCH_2CONH_2$ | 256–7 |
| 26 | 6-Cl | 4-Cl–$C_6H_4$ | $N(CH_3)_2$ | 230 |
| 27 | 6-Cl | 4-Cl–$C_6H_4$ | $N(C_2H_5)_2$ | 149 |
| 28 | 6-Cl | 4-Cl–$C_6H_4$ | N(n–$C_3H_7$)$_2$ | 140–1 |
| 29 | 6-Cl | 4-Cl–$C_6H_4$ | 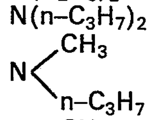 | 160 |
| 30 | 6-Cl | 4-Cl–$C_6H_4$ | 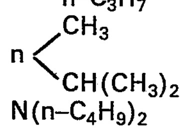 | 185–6 |
| 31 | 6-Cl | 4-Cl–$C_6H_4$ | N(n–$C_4H_9$)$_2$ | 149–150 |
| 32 | 6-Cl | 4-Cl–$C_6H_4$ | 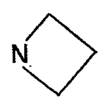 | 243–5 |
| 33 | 6-Cl | 4-Cl–$C_6H_4$ | 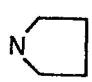 | 219–220 |
| 34 | 6-Cl | 4-Cl–$C_6H_4$ | 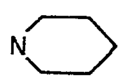 | 208–9 |
| 35 | 6-Cl | 4-Cl–$C_6H_4$ | 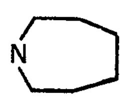 | 190–2 |
| 36 | 6-Cl | 4-Cl–$C_6H_4$ | 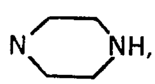, 2HCl | >300 |
| 37 | 6-Cl | 4-Cl–$C_6H_4$ | 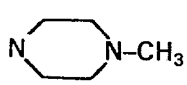 | 204–6 |
| 38 | 6-Cl | 4-Cl–$C_6H_4$ | 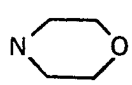 | 262 |

Tableau (suite 2)

| Composé | Y | Z | $NR_1R_2$ | F(°C) |
|---|---|---|---|---|
| 39 | 6-Cl | $4-Cl-C_6H_4$ | 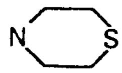 | 239–241 |
| 40 | 6-Cl | $4-Cl-C_6H_4$ | 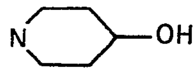 | 270 |
| 41 | $6-CH_3$ | $4-Cl-C_6H_4$ | $NHCH_3$ | 261–2 |
| 42 | $6-CH_3$ | $4-Cl-C_6H_4$ | $NHC_2H_5$ | 224–5 |
| 43 | $6-CH_3$ | $4-Cl-C_6H_4$ | $NH-CH_2CH_2OH$ | 246 |
| 44 | $6-CH_3$ | $4-Cl-C_6H_4$ | $N(CH_3)_2$ | 215 |
| 45 | $6-CH_3$ | $4-Cl-C_6H_4$ | $NH-CH_2-CH_2-Cl$ | 202 |
| 46 | $6-CH_3$ | $4-Cl-C_6H_4$ | 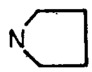 | 194 |
| 47 | 6-Cl | $C_6H_5$ | $NHCH_3$ | 276–7 |
| 48 | 6-Cl | $C_6H_5$ | $N(CH_3)_2$ | 192 |
| 49 | 6-Cl | $4-CH_3-C_6H_4$ | $NHCH_3$ | 277–8 |
| 50 | 6-Cl | $4-CH_3-C_6H_4$ | $N(CH_3)_2$ | 185–6 |
| 51 | 6-Cl | $4-CH_3O-C_6H_4$ | $NHCH_3$ | 273 |
| 52 | 6-Cl | $4-CH_3O-C_6H_4$ | $N(CH_3)_2$ | 166 |
| 53 | 6-Cl | $4-Br-C_6H_4$ | $NHC_2H_5$ | 287 |
| 54 | 6-Cl | $4-Br-C_6H_4$ | $N(C_2H_5)_2$ | 168 |
| 55 | 6-Cl | naphtyl-2 | 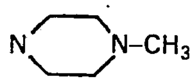 | 217–8 |
| 56 | 6-Cl | naphtyl-2 | 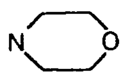 | 193–4 |
| 57 | 6-Cl | naphtyl-1 | $N(CH_3)_2$ | 187–8 |
| 58 | 6-Cl | $2-CH_3-C_6H_4$ | $NHCH_3$ | 175–6 |
| 59 | 6-Cl | $2-CH_3-C_6H_4$ | $NHC_2H_5$ | 161–2 |
| 60 | 6-Cl | $2-CH_3O-C_6H_4$ | $NHC_2H_5$ | 172–3 |
| 61 | 6-Cl | $3-Cl-C_6H_4$ | $NHC_2H_5$ | 215–6 |
| 62 | 6-Cl | $3-CH_3O-C_6H_4$ | $N(C_2H_5)_2$ | 98–9 |
| 63 | 6-Cl | $3-CH_3O-C_6H_4$ | 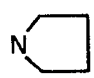 | 190 |
| 64 | 6-Cl | $3,4-Cl_2-C_6H_3$ | $N(CH_3)_2$ | 221–2 |
| 65 | 6-Cl | $3,4-(CH_3O)_2-C_6H_3$ | $N(CH_3)_2$ | 215 |
| 66 | 6-Cl | $3,4-(CH_3O)_2-C_6H_3$ | $N(n-C_3H_7)_2$ | 147 |
| 67 | $7-CH_3$ | $4-Cl-C_6H_4$ | $NHC_2H_5$ | 228 |
| 68 | $7-CH_3$ | $4-Cl-C_6H_4$ | $N(CH_3)_2$ | 206 |
| 69 | $8-CH_3$ | $4-Cl-C_6H_4$ | $NHCH_3$ | 234 |
| 70 | $8-CH_3$ | $4-Cl-C_6H_4$ | $N(C_2H_5)_2$ | 175,5 |
| 71 | 6-Cl | $4-F-C_6H_4$ | $N(CH_3)_2$ | 210 |
| 72 | 6-Cl | $4-F-C_6H_4$ | $N(n-C_4H_9)_2$ | 129 |
| 73 | $6-CH_3$ | $4-F-C_6H_4$ | $N(CH_3)_2$ | 195 |
| 74 | 6-Cl | $4-Br-C_6H_4$ | $N(CH_3)_2$ | 228–9 |
| 75 | $6-CH_3$ | $4-CH_3-C_6H_4$ | $N(CH_3)_2$ | 196 |
| 76 | $6-CH_3$ | $4-Cl-C_6H_4$ | $N(n-C_4H_9)_2$ | 116 |
| 77 | 6-Cl | $4-Cl-C_6H_4$ | 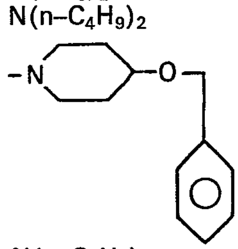 | 152 |
| 78 | H | $4-Cl-C_6H_4$ | $N(n-C_3H_7)_2$ | 136 |
| 79 | H | $4-Cl-C_6H_4$ | $N(n-C_4H_9)_2$ | 105 |

Tableau (suite 3)

| Composé | Y | Z | $NR_1R_2$ | F(°C) |
|---|---|---|---|---|
| 80 | 6-Cl | (4-Cl–$C_6H_4$ | $N(n{-}C_5H_{11})_2$ | 92–3 |
| 81 | 6-$CH_3$ | 4-$CH_3$–$C_6H_4$ | $NHCH_3$ | 187 |
| 82 | 6-$CH_3$ | 4-$CH_3$–$C_6H_4$ | $NHC_2H_5$ | 184 |
| 83 | 6-$CH_3$ | 4-$CH_3C_6H_4$ | 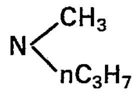 | 108 |
| 84 | 6-$CH_3$ | 4-$CH_3C_6H_4$ | $N(n{-}C_3H_7)_2$ | 115 |
| 85 | 6-$CH_3$ | 4-$CH_3$–$C_6H_4$ | 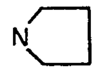 | 168 |
| 86 | 6-$CH_3$ | 4-Cl–$C_6H_4$ | $NHCH_2CF_3$ | 239 |
| 87 | 6-$CH_3$ | 4-Br–$C_6H_4$ | $NHC_2H_5$ | 232–4 |
| 88 | 6-$CH_3$ | 4-Br–$C_6H_4$ | $N(CH_3)_2$ | 203,5–205 |
| 89 | 6-$CH_3$ | 4-Br–$C_6H_4$ | $N(n{-}C_3H_7)_2$ | 138–9 |
| 90 | 6-$CH_3$ | 4-Br–$C_6H_4$ | 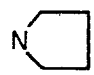 | 195,5–197 |
| 91 | 6-$CH_3$ | 4-$CH_3O$–$C_6H_4$ | $N(CH_3)_2{\cdot}CH_3SO_3H$ | 230–2 |
| 92 | 6-$CH_3$ | 4-$CH_3S$–$C_6H_4$ | $N(CH_3)_2{\cdot}CH_3SO_3H$ | 209 |
| 93 | 6-$CH_3$ | 4-$CH_3SO_2$–$C_6H_4$ | $N(CH_3)_2$ | 227–9 |
| 94 | 6-$CH_3$ | 4-$NO_2$–$C_6H_4$ | $NHC_2H_5$ | 268–270 |
| 95 | 6-$CH_3$ | 4-$NO_2$–$C_6H_4$ | $N(CH_3)_2$ | 262–3 |
| 96 | 6-$CH_3$ | 4-t-$C_4H_9$–$C_6H_4$ | $N(CH_3)_2$ | 199–200 |
| 97 | 6-Cl | 4-Cl–$C_6H_4$ | 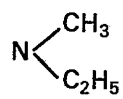 | 173 |
| 98 | 6-$CH_3$ | 3-$CH_3$–$C_6H_4$ | $(N(CH_3)_2$ | 157–158 |

Les composés de l'invention ont été soumis à des essais pharmacologiques qui ont montré leurs intéressantes propriétés pharmacologiques dans divers domaines.

La toxicité des composés a été déterminée chez las souris par voie intrapéritonéale.

La DL 50 va de 500 à 1000 mg/kg.

L'activité anxiolytique a été déterminée selon le «eating test» (Stephens, R.J. (1973) Brit. J. Pharmac., 49, 146 P).

Dans ce test, les doses qui augmentent la consommation alimentaire des souris varient de 0,1 à 10 mg/kg, i.p.

L'activité des composés dans le domaine de la circulation cérébrale ont été déterminée dans le test de l'hypoxie hypobare.

Des souris de souche CD1 sont maintenues dans une atmosphère appauvrie en oxygène, par réalisation d'un vide partiel (190 mm de mercure correspondant à 5,25% d'oxygène).

Le temps de survie des animaux est noté. Ce temps est augmenté par les agents capables de favoriser l'oxygénation tissulaire et en particulier cérébrale. Les composés étudiés sont administrés, à plusieurs doses, par voie intrapéritonéale, 10 minutes avant l'essai. Les pourcentages d'augmentation du temps de survie par rapport aux valeurs obtenues chez les animaux témoins sont calculés. La dose active moyenne (DAM), dose qui augmente le temps de survie de 100% est déterminée graphiquement. La DAM va de 0,3 à 32 mg/kg i.p.

L'activité anticonvulsivante a été déterminée selon le test de l'antagonisme vis à vis de la mortalité induite par la bicuculline chez la souris (Worms, P., Depoortere, H. and Lloyd, K.G. (1979) Life Sci., 25, 607–614). Les produits à étudier sont injectés par voie intrapéritonéale, 30 mn avant la bicuculline (0,9 mg/kg i.v.). Le critère retenu pour ce test étant la léthalité, les pourcentages de mortalité sont notés pour chaque lot, 2 heures après administration de la bicuculline (lot témoin: 100% mortalité).

Pour chaque produit la dose active 50% (DA 50 ou dose protégeant 50% d'animaux des effets léthaux de la bicuculline) est déterminée graphiquement. La DA 50 des composés de l'invention varie entre 0,3 et 30 mg/kg par voie i.p.

L'activité sédative ou hypnotique a été déterminée en observant l'action des composés sur l'ECoG du rat curarisé ainsi que sur les états de veille-sommeil chez le rat et chez le chat implanté libres (Depoortere, H., Rev. E.E.G. Neurophysiol., (1980) 10, 3, 207–214; Da Costa, L.M., Depoortere, H. et Naquet, R. Rev. E.E.G. Neurophysiol., (1977), 7, 2, 158–164). Chez le rat curarisé, les produits à étudier ont été injectés par voie intrapéritonéale ou orale aux doses croissantes de 0,1 à 30 mg/kg. Ils induisent des tracés de sommeil à partir des doses allant de 0,1 à 10 mg/kg i.p. ou

p.o. Chez le rat implanté libre, les produits à étudier ont été injectés par voie intrapéritonéale ou orale, à dose unique allant de 1 à 10 mg/kg. Ils réduisent, à ces doses, la durée totale de l'éveil de 13 à 44% sans modification importante de la durée totale du sommeil paradoxal; certains produits augmentant même la durée totale de cette phase de sommeil. Chez le chat implanté libre, les produits à étudier ont été injectés par voie intrapéritonéale ou orale à la dose unique de 10 mg/kg. Ils augmentent transitoirement l'éveil après l'injection de façon concomittante à une agitation de type benzodiazépinique, et réduisent la durée totale du sommeil paradoxal de 40 à 100%. Toutefois, certains produits augmentent la durée totale du SPOL (sommeil à ondes lentes avec phénomènes phasiques: pointes P.G.O.) de l'ordre de 50%.

Les résultats de ces différents tests montrent que les composés de l'invention possèdent des propriétés anxiolytiques, antianoxiques, inductrices de sommeil, hypnotiques et anticonvulsivantes; les composés de l'invention sont utiles pour le traitement des états d'anxiétés, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, ainsi que pour le traitement des absences dues à des traumatismes crâniens et pour le traitement des encéphalopathies métaboliques.

Les composés de l'invention peuvent être présentés sous toute forme appropriée pour l'administration par voie orale, ou parentérale, par exemple sous forme de comprimés, de dragées, de gélules, de solutions buvables ou injectables etc. avec tout excipient approprié.

La posologie quotidienne peut aller de 0,5 à 2000 mg.

**Revendications pour les états contractants: BE, CH, DE, FR, GB, IT, LI, LU, NL, SE**

1. Imidazo[1,2-a]pyridines répondant à la formule (I)

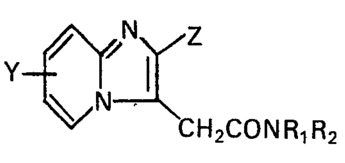

(I)

dans laquelle

Y représente un atome d'hydrogène ou d'halogène ou un radical $C_{1-4}$alkyle,

Z représente un radical naphtyle ou un radical

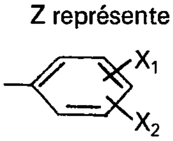 dans lequel $X_1$ et $X_2$ sont chacun indépendamment l'un de l'autre, un atome d'hydrogène ou d'halogène, un radical $C_{1-4}$alcoxy, un radical $C_{1-6}$alkyle, le groupe $CF_3$, $CH_3S$, $CH_3SO_2$ ou $NO_2$,

$R_1$ et $R_2$ représentent chacun indépendamment l'un de l'autre soit un atome d'hydrogène, soit un radical $C_{1-5}$alkyle droit ou ramifié pouvant porter un ou plusieurs atomes d'halogène, un radical hydroxy, $N(C_{1-4}alkyl)_2$, carbamoyl ou $C_{1-4}$alcoxy, soit le radical allyle, soit le radical propargyle, soit un radical $C_{3-6}$cycloalkyle, soit le radical benzyle, soit le radical phényle,

$R_1$ et $R_2$ ne pouvant être tous deux des atomes d'hydrogène ou bien

$NR_1R_2$ représentent ensemble un hétérocycle comportant de 3 à 6 atomes de carbone ou un hétérocycle de formule 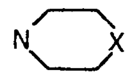 dans laquelle X est O,S,CHOR' ou N–R, R' étant un atome d'hydrogène ou le radical benzyle et R étant un atome d'hydrogène, un radical $C_{1-4}$alkyle ou le radical phényle pouvant porter un radical méthoxy ou un atome d'halogène, ainsi que leurs sels d'addition aux acides pharmacologiquement acceptables.

2. Dérivés selon la revendication 1, dans lesquels $R_1$ et $R_2$ sont tous deux des radicaux alkyles.

3. Dérivés selon la revendication 1, dans lesquels Y est en position 6.

4. Dérivés selon la revendication 3, dans lesquels Y est un atome de chlore ou le radical méthyle.

5. Dérivés selon la revendication 1, dans lesquels Z est le radical $X_1$ 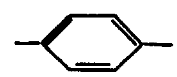 ; $X_1$ ayant la signification donnée dans la revendication 1.

6. Dérivés selon la revendication 5, dans lesquels Z est le radical Hal

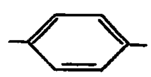 où $CH_3$ 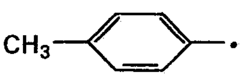.

7. Dérivés selon la revendication 1, dans lesquels $R_1$ et $R_2$ sont des radicaux alkyles, Y est un atome d'halogène ou le radical méthyle en position 6 et Z est le radical $X_1$ 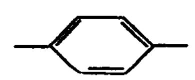 où $X_1$ est un atome d'halogène ou le radical méthyle.

8. Le méthyl-6 N,N-diméthyl (méthyl-4 phényl)-2 imidazo [1,2-a]pyridine-3-acétamide et ses sels pharmaceutiquement acceptables.

9. Le méthyl-6 N,N-diméthyl (chloro-4 phényl)-2 imidazo [1,2-a]pyridine-3-acétamide et ses sels pharmaceutiquement acceptables.

10. Le chloro-6 N,N-di-n-propyl (chloro-4 phényl)-2 imidazo [1,2-a]pyridine-3-acétamide et ses sels pharmaceutiquement acceptables.

11. Le méthyl-6 N,N-diméthyl (méthylthio-4 phényl)-2 imidazo [1,2-a]pyridine-3-acétamide et ses sels pharmaceutiquement acceptables.

12. Procédé de préparation des composés selon la revendication 1, procédé caractérisé en ce que l'on transforme un nitrile de formule (II)

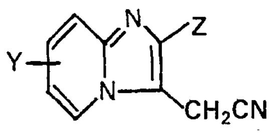

(II)

en amide primaire (III)

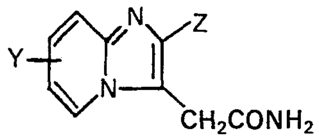

(III)

puis on transforme cet amide en acide (IV)

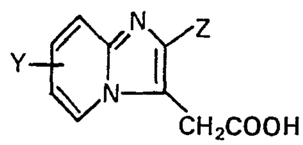

(IV)

et enfin on amidifie l'acide (IV)

13. Médicament caractérisé en ce qu'il contient un composé tel que spécifié dans l'une quelconque des revendications 1 à 11.

14 Composition pharmaceutique caractérisée en ce qu'elle contient un composé tel que spécifié dans l'une quelconque des revendications 1 à 11 en association avec tout excipient approprié.

**Revendication pour l'état contractant: AT**

Procédé de préparation des imidazo [1,2-a]pyridines répondant à la formule (I)

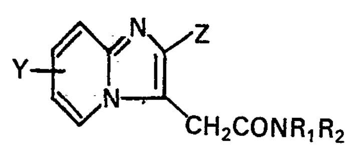

(I)

dans laquelle

Y représente un atome d'hydrogène ou d'halogène ou un radical $C_{1-4}$ alkyle,

Z représente un radical naphtyle ou un radical

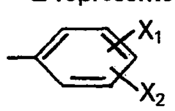

dans lequel $X_1$ et $X_2$ sont chacun indépendamment l'un de l'autre, un atome d'hydrogène ou d'halogène, un radical $C_{1-4}$ alcoxy, un radical $C_{1-6}$ alkyle, le groupe $CF_3$, $CH_3S$, $CH_3SO_2$ ou $NO_2$,

$R_1$ et $R_2$ représentent chacun indépendamment l'un de l'autre soit un atome d'hydrogène, soit un radical $C_{1-5}$ alkyle droit ou ramifié pouvant porter un ou plusieurs atomes d'halogène, un radical hydroxy $N(C_{1-4}\ alkyl)_2$, carbamoyl ou $C_{1-4}$ alcoxy, soit le radical allyle, soit le radical propargyle, soit un radical $C_{3-6}$ cycloalkyle, soit le radical benzyle, soit le radical phényle,

$R_1$ et $R_2$ ne pouvant être tous deux des atomes d'hydrogène ou bien

$NR_1R_2$ représentent ensemble un hétérocycle comportant de 3 à 6 atomes de carbone ou un hétérocycle de formule 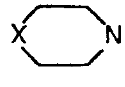 dans laquelle X est O,S,CHOR' ou N–R, R' étant un atome d'hydrogène ou le radical benzyle et R étant un atome d'hydrogène, un radical $C_{1-4}$ alkyle ou le radical phényle pouvant porter un radical méthoxy ou un atome d'halogène, ainsi que de leurs sels d'addition aux acides pharmaceutiquement acceptables, procédé caractérisé en ce que l'on transforme un nitrile de formule (II)

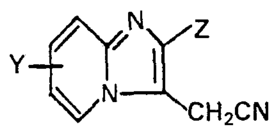

(II)

en amide primaire (III)

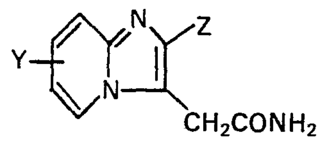

(III)

puis on transforme cet amide en acide (IV)

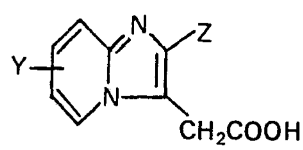

(IV)

et enfin on amidifie l'acide (IV)

**Patentansprüche für die Vertragsstaaten: BE, CH, DE, FR, GB, IT, LI, LU, NL und SE**

1. Imidazo[1,2-a]pyridine der Formel (I)

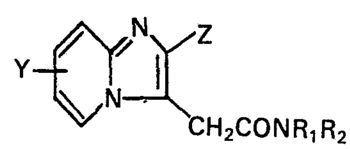

(I)

in der

Y ein Wasserstoffatom, ein Halogenatom oder eine $C_{1-4}$-Alkylgruppe,

Z eine Naphthylgruppe oder eine Gruppe der Formel 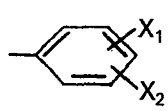 in der $X_1$ und $X_2$ unabhängig voneinander Wasserstoffatome oder Halogenatome, $C_{1-4}$-Alkoxygruppen, $C_{1-6}$-Alkylgruppen, $CF_3$-Gruppen, $CH_3S$-Gruppen, $CH_3SO_2$-Gruppen oder $NO_2$-Gruppen darstellen, und

$R_1$ und $R_2$ unabhängig voneinander jeweils entweder ein Wasserstoffatom oder eine geradkettige oder verzweigte $C_{1-5}$-Alkylgruppe, die eines oder mehrere Halogenatome, Hydroxylgruppen, $N(C_{1-4}\text{-}Alkyl)_2$-Gruppen, Carbamoylgruppen oder $C_{1-4}$-Alkoxygruppen tragen kann, oder eine Allylgruppe, oder eine Propargylgruppe, oder eine $C_{3-6}$-Cycloalkylgruppe, oder eine Benzylgruppe oder eine Phenylgruppe, mit der Massgabe bedeuten, dass $R_1$ und $R_2$ nicht beide jeweils Wasserstoffatome darstellen, oder

$NR_1R_2$ gemeinsam einen Heterozyklus mit 3 bis

6 Kohlenstoffatomen oder einen Heterozyklus der Formel 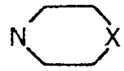 in der X, O, S, CHOR' oder N–R darstellt, worin R' für ein Wasserstoffatom oder eine Benzylgruppe und R für ein Wasserstoffatom oder eine $C_{1-4}$-Alkylgruppe oder eine Phenylgruppe, die eine Methoxygruppe oder ein Halogenatom tragen kann, stehen, bedeuten, sowie deren Additionssalze mit pharmazeutisch annehmbaren Säuren.

2. Derivate nach Anspruch 1, dadurch gekennzeichnet, dass $R_1$ und $R_2$ jeweils Alkylgruppen bedeuten.

3. Derivate nach Anspruch 1, dadurch gekennzeichnet, dass Y in der 6-Stellung steht.

4. Derivate nach Anspruch 3, dadurch gekennzeichnet, dass Y ein Chloratom oder eine Methylgruppe bedeutet.

5. Derivate nach Anspruch 1, dadurch gekennzeichnet, dass Z eine Gruppe der Formel $X_1$ 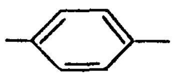 darstellt, in der $X_1$ die in Anspruch 1 angegebenen Bedeutungen besitzt.

6. Derivate nach Anspruch 5, dadurch gekennzeichnet, dass Z eine Gruppe der Formel Hal 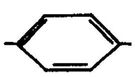 oder der Formel $CH_3$ 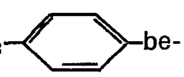 bedeutet.

7. Derivate nach Anspruch 1, dadurch gekennzeichnet, dass $R_1$ und $R_2$ Alkylgruppen, Y ein Halogenatom oder eine Methylgruppe in der 6-Stellung und Z eine Gruppe der Formel $X_1$ 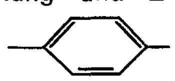, worin $X_1$ für ein Halogenatom oder eine Methylgruppe steht, bedeuten.

8. 6-Methyl-N,N-dimethyl-2-(4-methylphenyl)-imidazo[1,2-a]pyridin-3-acetamid und dessen pharmazeutisch annehmbare Salze.

9. 6-Methyl-N,N-dimethyl-2-(4-chlorphenyl)-imidazo[1,2-a]pyridin-3-acetamid und dessen pharmazeutisch annehmbare Salze.

10. 6-Chlor-N,N-di-n-propyl-2-(4-chlorphenyl)-imidazo[1,2-a]pyridin-3-acetamid und dessen pharmazeutisch annehmbare Salze.

11. 6-Methyl-N,N-dimethyl-2-(4-methylthiophenyl)-imidazo[1,2-a]pyridin-3-acetamid und dessen pharmazeutisch annehmbare Salze.

12. Verfahren zur Herstellung der Verbindungen nach Anspruch 1, dadurch gekennzeichnet, dass man ein Nitril der Formel (II)

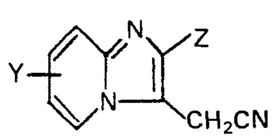 (II)

in ein primäres Amid (III)

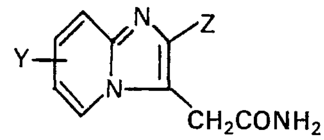 (III)

umwandelt und dann dieses Amid in die Säure (IV)

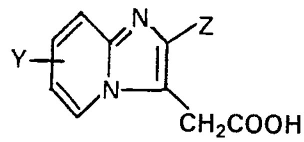 (IV)

umwandelt, wonach man die Säure (IV) in das Amid überführt.

13. Arzneimittel, dadurch gekennzeichnet, dass es eine Verbindung nach einem der Ansprüche 1 bis 11 enthält.

14. Pharmazeutische Zubereitung, dadurch gekennzeichnet, dass sie eine Verbindung nach einem der Ansprüche 1 bis 11 in Kombination mit irgendeinem geeigneten Hilfsstoff enthält.

**Patentanspruch für den Vertragsstaat: AT**

Verfahren zur Herstellung von Imidazo[1,2-a]pyridinen der Formel (I)

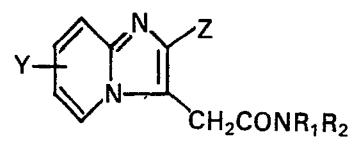 (I)

in der

Y ein Wasserstoffatom, ein Halogenatom oder eine $C_{1-4}$-Alkylgruppe,

Z eine Naphthylgruppe oder eine Gruppe der Formel 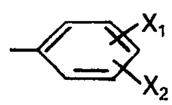 in der $X_1$ und $X_2$ jeweils unabhängig voneinander für Wasserstoffatome, Halogenatome, $C_{1-4}$-Alkoxygruppen, $C_{1-6}$-Alkylgruppen, $CF_3$-Gruppen, $CH_3S$-Gruppen, $CH_3SO_2$-Gruppen oder $NO_2$-Gruppen stehen, und

$R_1$ und $R_2$ unabhängig voneinander jeweils ein Wasserstoffatom oder eine geradkettige oder verzweigte $C_{1-5}$-Alkylgruppe, die eines oder mehrere Halogenatome, Hydroxylgruppen, $N(C_{1-4}\text{-Alkyl})_2$-Gruppen, Carbamoylgruppen oder $C_{1-4}$-Alkoxygruppen tragen kann, oder eine Allylgruppe, oder eine Propargylgruppe, oder eine $C_{3-6}$-Cycloalkylgruppe, oder eine Benzylgruppe oder eine Phenylgruppe mit der Massgabe bedeuten, dass $R_1$ und $R_2$ nicht jeweils beide Wasserstoffatome darstellen, oder

$NR_1R_2$ gemeinsam einen Heterozyklus mit 3 bis 6 Kohlenstoffatomen oder einen Heterozyklus der Formel 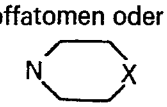 worin X, O, S, CHOR' oder N–R darstellt, worin R' für ein Wasserstoffatom oder eine Benzylgruppe und R für ein Was-

serstoffatom, eine $C_{1-4}$-Alkylgruppe oder eine Phenylgruppe, die eine Methoxygruppe oder ein Halogenatom tragen kann, stehen, bedeuten, sowie ihrer Additionssalze mit pharmazeutisch annehmbaren Säuren, dadurch gekennzeichnet, dass man ein Nitril der Formel (II)

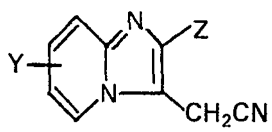

(II)

in das primäre Amid (III)

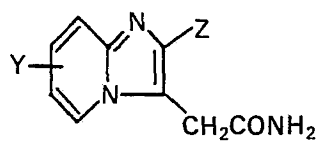

(III)

umwandelt und dann dieses Amid in die Säure (IV)

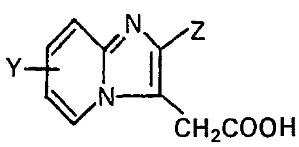

(IV)

umwandelt, wonach man die Säure (IV) in das Amid überführt.

**Claims for the contracting states: BE, CH, DE, FR, GB, IT, LI, LU, NL, SE.**

1. Imidazo[1,2-a]pyridines of the formula:

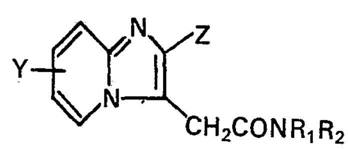

(I)

in which

Y represents a hydrogen or halogen atom or a $C_{1-4}$ alkyl radical,

Z represents a naphthyl radical or a radical

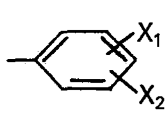

, in which each of $X_1$ and $X_2$ independently of one another is a hydrogen or halogen atom, a $C_{1-4}$ alkoxy radical, a $C_{1-6}$ alkyl radical or $CF_3$, $CH_3S-$, $CH_3SO_2-$ or $-NO_2$, and each of $R_1$ and $R_2$ independently of one another represents either a hydrogen atom, or a straight or branched $C_{1-5}$ alkyl radical which is unsubstituted or substituted by one or more halogen atoms, hydroxyl, $-N(C_{1-4}alkyl)_2$, carbamoyl or $C_{1-4}$ alkoxy radicals, or an allyl radical, or a propargyl radical, or a $C_{3-6}$ cycloalkyl radical, or a benzyl radical, or a phenyl radical, not both $R_1$ and $R_2$ being hydrogen, or $-NR_1R_2$ represents a heterocyclic ring containing from 3 to 6 carbon atoms, or a heterocyclic ring of the formula 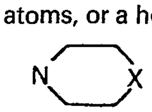 , in which X is O, S, CHOR' or N-R, R' being hydrogen or benzyl and R being hydrogen, a $C_{1-4}$ alkyl radical, or phenyl which is unsubstituted or substituted by methoxy or a halogen atom, and their addition salts with pharmacologically acceptable acids.

2. Derivatives according to claim 1, in which $R_1$ and $R_2$ are both alkyl radicals.

3. Derivatives according to claim 1 or 2, in which Y is in the 6-position.

4. Derivatives according to claim 3, in which Y is chlorine or methyl.

5. Derivatives according to claim 1, in which Z is the radical $X_1$ 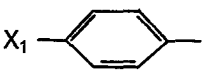, where $X_1$ has the meaning given in claim 1.

6. Derivatives according to claim 5, in which Z is the radical 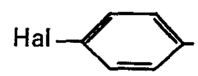 or 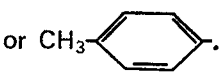.

7. Derivatives according to claim 1 in which $R_1$ and $R_2$ are both alkyl, Y is halogen or methyl in the 6-position, and Z is the radical $X_1$ 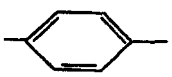 in which $X_1$ is halogen or methyl.

8. The 6-methyl N,N-dimethyl 2-(4-methylphenyl) imidazo [1,2-a] pyridine-3- acetamide and its pharmaceutically acceptable salts.

9. The 6-methyl N,N-dimethyl 2-(4-chlorophenyl) imidazo [1,2-a] pyridine-3- acetamide and its pharmaceutically acceptable salts.

10. The 6-chloro N,N-di-n propyl 2-(4-chlorophenyl) imidazo [1,2-a] pyridine-3-acetamide and its pharmaceutically acceptable salts.

11. The 6-methyl N,N-dimethyl 2-(4-methylthiophenyl) imidazo [1,2-a] pyridine-3-acetamide and its pharmaceutically acceptable salts.

12. Process for the preparation of a compound according to claim 1, which comprises hydrolysing a nitrile of the formula:

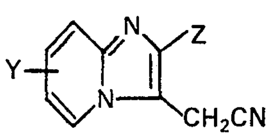

(II)

to a primary amide of formula:

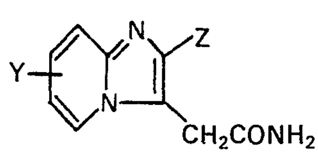

(III)

and then to an acid of formula:

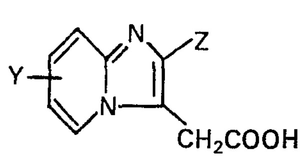

(IV)

and then converting the said acid into an amide of formula I as defined in claim 1.

13. A drug comprising a compound as specified in any one of claims 1 to 11.

14. A pharmaceutical composition, comprising a compound as specified in any one of claims 1 to 11, in association with a suitable excipient.

**Claim for the contracting state: AT**

Process for the preparation of a compound having the formula

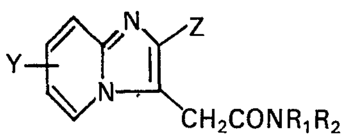

(I)

in which

Y represents a hydrogen or halogen atom or a $C_{1-4}$ alkyl radical,

Z represents a naphthyl radical or a radical

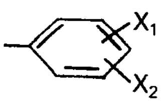

, in which each of $X_1$ and $X_2$ independently of one another is a hydrogen or halogen atom, a $C_{1-4}$ alkoxy radical, a $C_{1-6}$ alkyl radical or $CF_3$, $CH_3S-$, $CH_3SO_2-$ or $-NO_2$, and each of $R_1$ and $R_2$ independently of one another represents either a hydrogen atom, or a straight or branched $C_{1-5}$ alkyl radical which is unsubstituted or substituted by one or more halogen atoms, hydroxyl, $-N(C_{1-4}alkyl)_2$, carbamoyl or $C_{1-4}$ alkoxy radicals, or an allyl radical, or a propargyl radical, or a $C_{3-6}$ cycloalkyl radical, or a benzyl radical, or a phenyl radical, not both $R_1$ and $R_2$ being hydrogen, or $-NR_1R_2$ represents a heterocyclic ring containing from 3 to 6 carbon atoms, or a heterocyclic ring of the formula 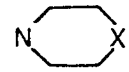 , in which X is O, S, CHOR' or N–R, R' being hydrogen or benzyl and R being hydrogen, a $C_{1-4}$ alkyl radical, or phenyl which is unsubstituted or substituted by methoxy or a halogen atom and of their addition salts with pharmacologically acceptable acids, process which comprises hydrolysing a nitrile of the formula

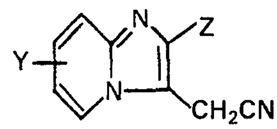

(II)

to a primary amide of formula:

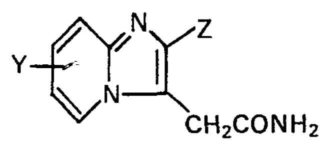

(III)

and then to an acid of formula:

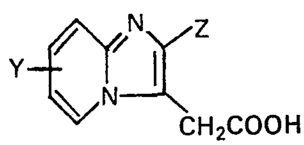

(IV)

and then converting the said acid into an amide of formula I as defined in claim 1, and eventually, converting the compound I into an addition salt with a pharmaceutically acceptable acid.